# EUROPEAN PATENT SPECIFICATION

(11) **EP 3 017 301 B1**
(45) Date of publication and mention of the grant of the patent: **12.12.2018**
(21) Application number: 14744478.0
(22) Date of filing: 02.07.2014
(51) Int. Cl.: G01N 33/50

(54) **MICROTISSUES**
MIKROGEWEBE
MICRO-TISSUS

(30) Priority: 02.07.2013 EP 13003337
(43) Date of publication of application: 11.05.2016
(73) Proprietor: ETH Zurich, 8092 Zurich (CH)
(72) Inventor: ZENOBI-WONG, Marcy, CH-8049 Zürich (CH); YANG, Yuan, Toronto, ON M4Y1R5 (CA); GROTH, Thomas, 10439 Berlin (DE); MILLAN, Christopher, Zürich 8037 (CH); MIRANDA-NIEVES, David, Cambridge, Massachusetts 02139 (US)
(74) Representative: Bühler, Dirk
(86) International application number: PCT/EP2014/064017
(87) International publication number: WO 2015/000933

(56) References cited:
- WO-A1-2014/032748
- HONGZHI ZHOU ET AL: "The fast release of stem cells from alginate-fibrin microbeads in injectable scaffolds for bone tissue engineering", BIOMATERIALS, ELSEVIER SCIENCE PUBLISHERS BV., BARKING, GB, vol. 32, no. 30, 19 June 2011 (2011-06-19) , pages 7503-7513, XP028261576, ISSN: 0142-9612, DOI: 10.1016/J.BIOMATERIALS.2011.06.045 [retrieved on 2011-06-27]
- HANWEI ZHANG ET AL: "In Situ Gelable Interpenetrating Double Network Hydrogel Formulated from Binary Components: Thiolated Chitosan and Oxidized Dextran", BIOMACROMOLECULES, vol. 12, no. 5, 9 May 2011 (2011-05-09), pages 1428-1437, XP055086079, ISSN: 1525-7797, DOI: 10.1021/bm101192b
- Lauren Kimlin ET AL: "3D in vitro tissue models and their potential for drug screening", Expert opinion on drug discovery, vol. 8, no. 12, 22 October 2013 (2013-10-22), pages 1455-1466, XP055428103, London, GB ISSN: 1746-0441, DOI: 10.1517/17460441.2013.852181

## Description

This disclosure relates to microtissues adapted for a variety of uses, including forming the basis of pharmaceutical screens.

Zhou et al. (Biomaterials (2011), 32, 7503-7513) describes fast-degradable alginate-fibrin microbeads with human umbilical cord mesenchymal stem cell (hUCMSC) encapsulation. Zhang et al. (Biomacromolecules (2011), 12, 1428-1437) describes in situ gelable interpenetrating double-network hydrogels composed of thiolated chitosan and oxidized dextran.

It has been proposed to make microtissues for use, for example, as pharmaceutical screens using fluorescent detectors, for example, reagent-based fluorescent assay or cells genetically modified to express a fluorescent promoter reporter. Traditionally, these have been two-dimensional surfaces. It is recognised that three-dimensional surfaces would be better in this regard (see, for example, Kisaalita WS. "3D cell-based biosensors in drug discovery programs: Microtissue engineering for high throughput screening": CRC Press; 2010.). Such 3-D structures also have the potential to create building blocks for use in tissue engineering (see, for example, Elbert DL. "Bottom-up tissue engineering. Current opinion in biotechnology". 2011;22:674-80 and Fennema E, Rivron N, Rouwkema J, van Blitterswijk C, de Boer J. "Spheroid culture as a tool for creating 3D complex tissues". Trends in biotechnology. 2013.)

The methods currently available to the art for the creation of such include centrifugation, hanging drop culture, gyratory shakers or spinner flasks. All of these methods, however, depend on the formation of cell-cell and cell-matrix adhesions based on receptors of the cadherin, ICAM and integrin families and thus require culture times on the order of hours, sometimes days, before a robust microtissue is formed. If the cell adhesion receptors are missing on the cell surface, as is often the case of cells which have been cryopreserved, the formation of the spheroid fails.

It has now been found that it is possible to make a desirable 3-D microtissue in as little as a matter of a few minutes. There is therefore provided a method of making a three-dimensional microtissue comprising the reaction of at least two cytocompatible polymer solutions, each polymer comprising complementary reactive groups capable of spontaneous reaction, at least one of which solutions additionally contains cells.

There is additionally provided a three-dimensional microtissue comprising a crosslinked composition of at least two cytocompatible polymers and cells, the crosslinking being provided by complementary reactive groups of two types, one of these types being present on each one of the cytocompatible polymers.

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of' is considered to be a preferred embodiment of the term "comprising of'. If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" or "(i)", "(ii)", "(iii)", "(iv)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" or "(i)", "(ii)", "(iii)", "(iv)" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein, as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

The cytocompatible polymers for use in this method may be any suitable polymers with the necessary cytocompatibility, that is, their presence is not harmful to cells. They may be natural or synthetic materials. The necessary complementary reactive groups may be already present on the polymers, or the polymers may be modified to include such groups. This is within the skill of the art in every case.

Typical non-limiting examples of natural polymers include alginate, alginate sulfate, chondroitin sulfate, dermatin sulfate, hyaluronic acid, cellulose, dextran, poly-l-lysine, chitosan, gelatin, silk and collagen.

Typical non-limiting examples of synthetic polymers include polymers, or polymers derived from, poly ethylene glycol, poly propylene glycol, polaxomers, poly oxazolines, poly ethylenimine, poly vinyl alcohol, poly vinyl acetate, poly methyl vinyl ether-co-maleic anhydride, poly lactide, poly N-isopropylacrylamide, poly glycolic acid, poly methylmethacrylate, poly acrylamide, poly acrylic acid, polyallylamine.

The complementary reactive groups are those that will react spontaneously on being brought together. Many such reactive pairs are known to the art. Non-limiting examples of suitable complementary groups include *inter alia* those that result in Michael addition, disulfide bond formation, catechol-initiator polymerization, and enzyme-mediated crosslinking.

A typical complementary pair of reactive groups is the aldehyde/amine pairing that can lead to a Schiff base linkage. For example, aldehyde groups may be generated on biopolymers such as alginate, alginate sulfate, chondroitin sulfate, dermatin sulfate, hyaluronic acid, cellulose and dextran by chemical oxidation with suitable reagents, such as sodium periodate, sodium (meta) periodate and hydrogen peroxide. The equivalent amine-containing polymers may include proteins and peptides with a high percentage of lysine like chitosan, gelatin, silk, and collagen and synthetic polymers containing an amine group in their main or side chains like polyallylamine and polyethylenimine. One may also chemically modified Chitosan. Chitosan naturally has primary amines in its monomer unit, but these prevent its solubility at pH 7.4 (pure chitosan can only be solubilized in acid). By introducing succinyl groups to some of the amines , one can break up these crystallinity domains and permit its solubility at physiological pH. It is important to balance N-substitution with succinyl groups to overcome the solubility barrier, but to leave behind enough of the amines so that they are available for Schiff base crosslinking later on. One preferred degree of substitution of amine groups by succinyl groups lies within the range of about 25 to about 45%, preferably within about 30 to about 40% and more preferably around about 35%.

Tests for the presence of aldehydes, and Schiff bases prepared by this reaction are well known in fields of chemistry as diverse as perfumery, dyestuffs and liquid crystals. However, it has never been seen as a means of crosslinking tissue to form a scaffold material.

As mentioned, the necessary reactive groups may be there naturally, for example, the amine functionality of amino acids. This may also be true of aldehyde or ketone functionality, but it may also be needed to be provided by surface modification. For example, a tissue may be modified by oxidation to provide the necessary groups or these groups attached with chemical linker molecules. Another possibility is to use a polymer that already contains the necessary groups.

Another possible complementary reaction may be thiol Michael addition reactions. In this case, thiol groups, which may be naturally present, for example, in amino acids such as cysteine, or introduced chemically may be reacted with Michael acceptors including acrylate esters, acrylonitrile, acrylamides, maleimides, alkyl methacrylates, cyanoacrylates and vinyl sulfones.

In the specific example of the Michael addition, this may be achieved, for example, by the reduction of disulfide bonds in tissue and tissue surfaces with tris(2-carboxyethyl)-phosphine TCEP to introduce free sulfhydryl groups. This can also be achieved by the coupling of polymers with dithiobispropionic hydrazide (DTPH) with EDC followed by reduction to generate the free thiol, the conversion of amines to free thiols by 2-iminothiolane (Traut's Reagent), thiolation of proteins by N-succinimidyl S-acetylthio-acetate (SATA) and the conversion of oxidized glycosaminoglycans to free thiols by 2-acetamido-4-mercaptobutyric acid hydrazide (AMBH): The free sulfhydryls can participate in scaffold formation and adhesion via reaction with Michael-type acceptors including, but not limited to, acrylate esters, acrylonitrile, acrylamides, maleimides, alkyl methacrylates, cyanoacrylates and vinyl sulfones.

In the specific example of enzymatic crosslinking, crosslinking is effected by an enzyme. This method relies on the presence of functional groups, which are present already on the polymers or which can be generated thereon. Typical non-limiting examples include horseradish peroxidase + hydrogen peroxide which catalyze the formation of covalent linkages between hydroxyphenols and transglutaminase which catalyzes the covalent bond between a free amine and carboxamide group of glutamine containing materials.

One will usually use two different polymers carrying the respective complementary reactive groups. However, one may also consider to use the same type of polymer, with the polymer of the first and second polymer solution carrying the respective complementary reactive groups.

In terms of the reactive groups, it is preferred to have a stoichiometry of about 1:1. However, this is not narrowly critical, and a variation of up to 20% is tolerable. In the case of the aldehyde-amine reaction, it is preferred to have an excess of amines, as this is more tolerated by the cells.

The cells, which may be present in one or both polymer solutions, may be selected from any suitable cells, depending on the desired end use of the microtissue. Such cells may be selected from the group comprising *inter alia* mesenchymal stem cells, embryonic stem cells, stromal cells, liver cells including Kupffer cells and macrophages, hepatocytes, neural cells, pancreatic cells, kidney cells, muscle cells, monocytes, endothelial cells, fibroblasts, epithelial cells, chondrocytes, and cancer cells. In terms of embryonic stem cells, induced pluripotent stem cells (iPS) may be preferred. The afore-mentioned cell types may preferably be human cells. Preferably, these cells, when used for the purposes of the present invention, are outside the human or animal body.

In the context of the present invention, three-dimensional microtissues obtained by the methods described herein are also referred to as "QuickStick" (QS). Particularly preferred microtissues may be using sChi and oxAlg.

In even more preferred embodiments, factors that induce and/or influence the development and/or differentiation of cells may be added to at least one of the polymer solutions to trigger development and/or differentiation into the desired end product. Such factors may be provided in single from or in the form of cocktails, i.e. mixtures. Such factors may be selected from the group comprising *inter alia* growth factors, cytokines, chemokines, polypeptides, enzymes, hormones, as well as receptors for any of the afore-mentioned factors. They may be particularly suitable to induce and/or at least contribute to the development and/or differentiation of e.g. mesenchymal stem cells, embryonic stem cells, progenitor cells into the desired "end product" cell type(s). Such factors may be provided by non-covalent association with or preferably covalent attachment to at least one of the polymer solutions. One may also use media of different compositions as factors for inducing development and/or differentiation of e.g. mesenchymal stem cells, embryonic stem cells, etc. into the desired "end product" cell type(s).

Examplary growth factors include *inter alia* morphogenetic protein families like IGF, FGFs, HGF, EGFs (e.g. EGF-1, VEGF), BMPs, (e.g. BMP-2, BMP-6, TGF-β3, and TGF-β1,. TGF-β and its different forms can be used to trigger development of stem cells into *inter alia* neural, epithithelial, endothelial, renal, hepatic, pancreatic, chondral, osteoblasts/osteoclasts, cardiac (cardiomyocytes), and myoblasts.

Exemplary polypeptides include *inter alia* RGD, GFOGER, tumor-specific polypeptides (e.g. pancreatic polypeptide, PP), and tumor antigens.

Examplary cytokines include *inter alia* TNF-a, interleukin family, interferons, erythropoietin, and thrombopoietin.

Examplary chemokines include *inter alia* CCL family, CXCL family, and CCR family.

One can also add the receptors for each of these bioactive molecules that might capture growth factors, cytokines, etc. secreted by the cells themselves within the microtissue

In Example 7, microtissues were made using MSCs in the 'standard' QuickStick method with TGF either loaded in sChi or oxAlg. As the expression of analyzed genes was greatly increased, this shows that the growth factor was bound to the QuickStick polymer network and effectively induced the differentiation of the stem cells.

The above discussed embodiments of using growth factors, cytokines etc have a number of attractive advantages. Stem cells for clinical therapies can now be isolated from a patient and assembled in a QuickStick microtissue that has all the necessary components to induce the differentiation of the cells into the desired phenotype. They can be implanted at a site of injury or disease and will undergo differentiation within the body to heal or cure the injured tissue. Alternatively, *in vitro* experiments are possible using QuickStick microtissues loaded with different induction molecules for differentiating stem cells along various lineages into cell phenotypes that can interact with each other. One can imagine a network of different microtissues that provide complex functions mimicking the different organ systems of the body, e.g. pancreas, liver, bladder, kidney, muscle, cardiac, neural, osteochondral, vasculature, connective tissue, as well as microtissues for metabolizing complicated drugs that need enzymes from both or various organs to be broken down. This goes towards the concept of having an 'organ on a chip' for screening analyses, or personalized medicine.

Thus, such microtissues such as pancreatic, hepatic, kidney, muscle, cardiac, neural, osteochondral, vasculature microtissues can be used screen for their influence of pharmaceutically active agents and to thus e.g. assess the metabolic fate of such pharmaceutically active agents. This is important information for drug development as it is e.g. known that drugs are differently metabolized by cytochrome P450 and as Example ... shows that expression of Cyp3a13 is different for microtissues of the present invention vs. three-dimensional microtissues of the prior art or two dimensional cells as obtainable in Petri dishes. As illustrated in Example 2, three dimensional microtissues made from mesenchymal stem cells, embryonic stem cells, induced pluripotent stem cells, etc. can be used to screen e.g. for agents that influence development and/or differentiation of a three-dimensional microtissue made from the group comprising mesenchymal stem cells, embryonic stem cells, induced pluripotent stem cells, etc. into e.g. downstream lineages.

For example, if a screen is desired, the cells may be genetically modified, so that they can fluoresce in the presence of compound hits. Fluorescence-based assays can be used to detect changes in proliferation, morphology, oxidative stress, cell signally, inflammation, cytotoxicity, genotoxicity. Detection can be performed on a plate reader, confocal microscope, two-photon microscopy to attain information on fluorescent intensity, distribution, polarization and variance with time. Preferred cells types are EGFP reporter cell lines.

There is therefore also provided a screen assay, comprising a microtissue as hereinabove described, in which the cells are adapted to fluoresce on exposure to a stimulus whose detection is desired.

The microtissues of this disclosure are prepared by first providing the cytocompatible polymers with the necessary complementary reactive groups, should they require modification, adding the desired cells to one or both of the solutions and then combining them. Many of the crosslinking reactions occur spontaneously, such that a microtissue is formed and can be handled with forceps after only a few minutes. The microtissues may be made in sheets, pellets or spheroids. They can be made directly in the wells of screening plates.

The size of the microtissue will be determined by the number of starting cells. The number of cells used generally ranges from about 1000 to about 1 million. The quantity of polymer to be used with the chosen quantity of cells is determined by the ability to provide a microtissue that is sufficiently cohesive to be manipulated, for example, picked up with forceps. The quantities of polymer needed will vary in each individual case, depending on polymer type and desired degree of cohesion in a given application, but this can be readily determined in each case by routine, non-inventive experimentation.

Further different types of cells or the same type of cells may be combined with the polymer solutions to obtain different patterning (see Example 5), e.g. by sequential or simultaneous pipetting, etc. The different patterning is useful for a number of applications to model complicated intercellular interactions with microtissue technology (renal networks of endothelial and epithelial cells, liver microtissues with hepatocytes and Kupffer cells, neural tissues with glial cells/astrocytes, co-cultures of stem cells and mature cell types which have been reported in the literature to have mutual benefits for maintaining cell phenotypes etc.).. For example, in such co-cultures stem cells have been found to secrete factors that promote activity in mature cells.

The methods of the invention further allow to obtain microtissues, e.g. for screening purposes of drugs in the time range of less than about 10 days, less than about 9 days, less than about 8 days, less than about 7 days, less than about 6 days, less than about 5 days, less than about 4 days, less than about 3 days, less than about 2 days, less than about 1 day, than about 23 hours, than about 22 hours, than about 21 hours, less than about 20 hours, less than about 19 hours, less than about 18 hours, less than about 17 hours, less than about 16 hours, less than about 15 hours, less than about 14 hours, less than about 13 hours, less than about 12 hours, less than about 11 hours, less than about 10 hours, less than about 9 hours, less than about 8 hours, less than about 7 hours, less than about 6 hours, less than about 5 hours, less than about 4 hours, less than about 3 hours, less than about 2 hours, or even less than about 1 hour.

Another advantage of the microtissues of the present invention is that they allow for adapting their mechanical stability by controlling e.g. the extent of cross-linking of the polymers.

In a particularly preferred embodiment the present invention relates to three-dimensional microtissues of hepatocytes which are obtainable by the methods described herein. Such microtissue may be used for screening the metabolic fate of pharmaceutically active agents and in particular their metabolism by e.g. enzymes of the cytochrome P450 family.

Another particularly preferred embodiment of the present invention relates to three-dimensional microtissues of mesenchymal stem cells, embryonic stem cells, induced pluripotents stem cells (iPS), etc. which are obtainable by the methods described herein and wherein the methods make use of, preferably covalently cross-linked, growth factor such TGF-β with TGF-b3 being an example and/or cytokines.

In both of these particularly preferred embodiments the microtissues may be made using sChi and oxAlg.

The method allows considerable versatility.
The possible uses of these microtissues include, but are not limited to
- Pharmaceutical screens
- Building blocks in tissue engineering.

The method is further described with reference to the following non-limiting examples, which depict particular embodiments.

The figures are as follows:
Figure 1 shows micrographs of the alcian staining of various microtissues. The size bars represent 200 microns (see Example 1 for details)
Figure 2A shows micrographs of a microtissue which has been immunohistochemically stained to detect collagen 2 protein using the monoclonal antibody II-II6B3 from the Development Studies Hybridoma Bank. Figure 2B is a control without any primary antibody. The size bars represent 200 microns
Figure 3 is a graph displaying glycosaminoglycan contents of pellets in chondrogenic media (C), non-chondrogenic media (NC), and microtissues prepared according to Example 1 in chondrogenic media (QS C).
Figure 4 is a micrograph of bovine chondrocytes which have been transduced with a collagen 2 promoter - GFP construct. The cells are suspended in 2% agarose for 7 days 30 and the collagen 2 reporter appears as green in several cells.

### Example 1:

To form microtissues using the Schiff linkage technique, human mesenchymal stem cells (hMSCS, Lonza Group Ltd, Basel, Switzerland) between p.6-p.9, human chondrocytes and human adipose derived stromal cells were suspended in a solution of 5 mg/mL sChi at a cell density of 20 x 10⁶ cells/mL. Drops of 5 µL each of oxidized alginate were prepared on plastic ring structures to confine the drop and 10 µL of the cells + S-Chi were pipetted into individual droplets of oxAlginate. The substrate was turned upside down and the reaction was carried out in an incubator at 37°C for 10 minutes. Microtissues were transferred to agarose coated-wells of a 96-well plate with fine-tipped forceps and cultured in chondrogenic media and growth factor free media. In parallel, the same cells were centrifuged to form micromass pellets (200k cells/each) for comparison and transferred to the same 96-well plate.

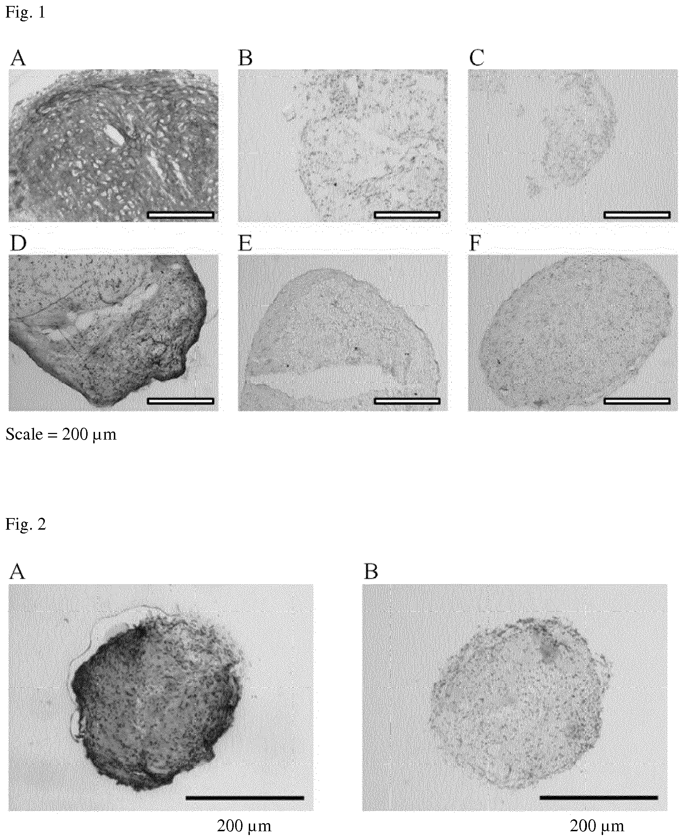

Figure 1 depicts the following possibilities:
Figure 1A - MSC-based microtissue formed via Schiff-base crosslinking using S-Chi and oxAlg with chondrogenic media.
Figure 1B - MSC-based microtissue formed via Schiff-base crosslinking using S-Chi and 20 oxAlg with non-chondrogenic media.
Figure 1C - MSC-based microtissue formed via centrifugation (art-standard method) with chondrogenic media
Figure 1D - Adipose stem cell-based microtissue formed via Schiff-base crosslinking using S-Chi and oxAlg with chondrogenic media.
Figure IE - Adipose stem cell-based microtissue formed via Schiff-base crosslinking using S-Chi and oxAlg with non-chondrogenic media.
Figure IF - Adipose stem cell-based microtissue formed centrifugation (art-standard method) with chondrogenic media.

Microtissues from Lonza MSCs formed via Schiff-base crosslinking using S-Chi and oxAlg demonstrated better chondrogenic induction than centrifuged pellets as indicated by alcian blue staining The intensity of the blue color shows the amount of negatively charged glycoaminoglycans produced by the cells, so in A there is much more of this molecule than in the art standard method. The practical implication is that the tissue generated looks much more like cartilage (which would also be stained dark blue)

Figure 2 shows a microtissue stained using a primary antibody against collagen 2, which is a marker of cartilage. An anti-mouse IgG1 secondary antibody and colorimetric reaction was used to visualize the localization of the protein. GAG or glycosaminoglycans are the other important part of cartilage.

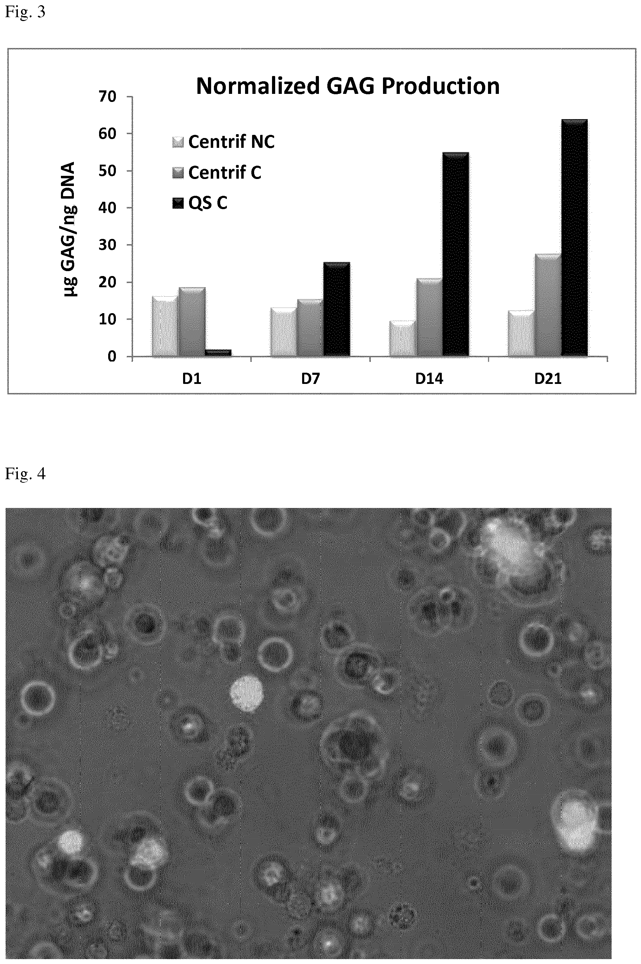

Figure 3 shows that the amount of GAG was the highest in the microtissue ("QS C"), as opposed to the centrifuged (Centrif) with chondrogenic (C) media and centrifuged with non-chondrogenic (NC) media respectively. Centrifugation is the art standard way of making microtissues.

Figure 2 shows that there is more blue staining in D compared to the art standard (however this induction was not as strong as with the bone marrow-derived MSCs).

The data indicate that crosslinked microtissues provide an immensely beneficial environment for differentiating stem cells into cartilage cells, versus the traditional technique of the art (i.e. centrifugation).

### Example 2: Use of collagen 2 promoter - GFP construct to detect individual cells which expression cartilage markers in 3D culture system.

Lentiviral chondrogenic promoter vectors comprised a transcriptional unit encoding for enhanced green fluorescent protein (EGFP) under the human collagen 2 promoter. A 6100 bp fragment of the human Col2 promoter was subcloned into pLVX-AcGFP-C1 by replacing the original CMV promoter. Subsequently, AcGFP was replaced by EGFP to create pLVX-Col(6100)-EGFP. To create pLVX-Col(-6100)-EGFP, a 1500 bp fragment and subsequently a 4600 bp fragment are excised from pKL7 and ligated into *corresponding* sites. EGFP fragment was amplified by PCR and integrated downstream the 6100 bp Col2 promoter. Correct orientation of the integrated EGFP was verified by restriction analysis.

*Transducing MSCs with Lenti-X viruses:* For transduction, the medium of the cells (seeded 12-16 hr prior transduction) was adjusted to accommodate the addition of virus and polybrene. Polybrene at a final concentration of 4 µl/ml is used to reduce the charge repulsion between the virus and the cell membrane. The lentiviral stock was diluted with medium to obtain the desired MOI and added to the cells for transduction. After 8 to 24 hr, virus-containing transduction medium was replaced with fresh growth medium. Cells are cultured and put into 3D culture to induce chondrogenic differentiation in the presence of chondrogenic medium (Dulbecco's Modified Eagle's Medium (DMEM, Gibco 41966), containing 1% Antibiotic-Antimycotic, 1% premixed IST, 100 µg/ml sodium pyruvate, 40 µg/ml L-proline, 50 µg/ml L-ascorbate-2-phosphate, 10 nM Dexamethasone, 10 ng/ml TGF-β3 (PeproTech) and 100 ng/ml BMP-2 (PeproTech))

As shown in Figure 4, in an environment which induces cartilage formation, the reporter lights up (these appear as white spots in Figure 4, green in reality). This experiment establishes the function of the collagen 2 promoter -GFP construct for detecting drugs and environmental conditions which induce cartilage matrix synthesis.

### Example 3: Speed, reliability and gene expression of microtissue formation by QuickStick

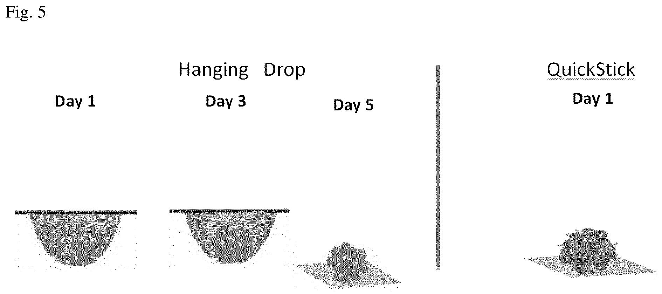

Primary hepatocytes were isolated from 6 donor mice and microtissues were formed with the cells either by the QuickStick method or by the classical hanging drop method (see e.g. Kelm et al., "Microscale tissue engineering using gravity-enforced cell assembly". Trends in biotechnology, 2004, see also Figure 5). For hanging drop, 20 thousand cells were pipetted per droplet into the lid of specialized well plate provided by Roche (see previous) in 15 uL droplets. The lids were turned upside down and the well plates were placed an incubator at 37°C and 5% CO2. Cells collect in the bottom of the droplet due to gravitational forces and, after 3-5 days, have formed into a microtissue due to cell-cell contacts. At 5 days, hanging drops were centrifuged down into wells of a conical 96-well plate at 50 xg for 30 seconds. For forming QuickStick microtissues, hepatocytes were suspended in a 5 mg/mL solution of N-succinyl chitosan (sChi) at 10,000 cells per µL. 2µL droplets of cells in sChi were mixed with 1µL droplets of 10 mg/mL oxidized alginate. Crosslinking between sChi and oxAlg was carried out in an incubator at 37°C for 20 minutes. Microtissues were then transferred to wells of a 96-well plate for culture.

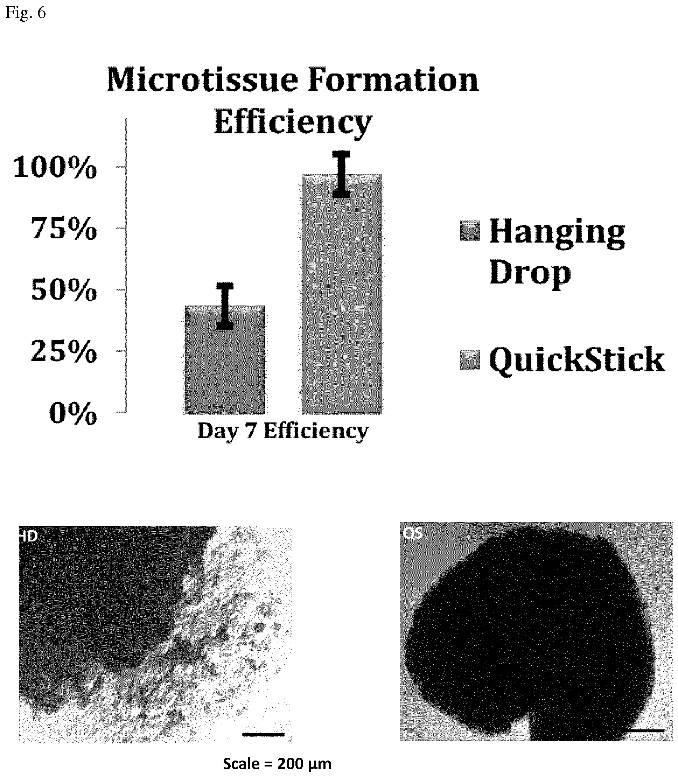

Microtissues according to the present invention can be obtained in less than a day and as short as within 15 minutes, while the conventional hanging drop method requires multiple days. On day 7, hepatocyte microtissues were imaged with brightfield microscopy to determine whether the microtissue structure remained in-tact. If individual cells could be seen outside of the central spheroid structure, the microtissue was deemed not to have formed. At day 7, nearly 100% of the QuickStick microtissues remained in-tact and robust (see picture under graph, HD=Hanging Drop, QS = QuickStick), while less than 50% of hanging drop microtissues had survived (see Figure 6).

Furthermore, function of hepatocytes in QuickStick versus hanging drop microtissues was assessed over a period of 5 days in *ex vivo* culture in media composed of D-MEM supplemented with 1% insulin-transferrin-selenium, 1% penicillin-streptomycin, and 20ng/mL hepatocyte growth factor. Expression of the Cyp3a13 gene, which is a member of the cytochrome P450 family responsible for the metabolism of over 50% of clinically active drugs, was evaluated at days 1, 3, and 5 during culture of hepatocyte microtissues. The expression level of the cyp3al3 gene was normalized to the value observed in freshly isolated hepatocytes (i.e. cells analyzed immediately after isolating from murine donors), which is the value taken to be the 100% expression level. The housekeeping gene used for calculating fold change in expression was mRPS29.

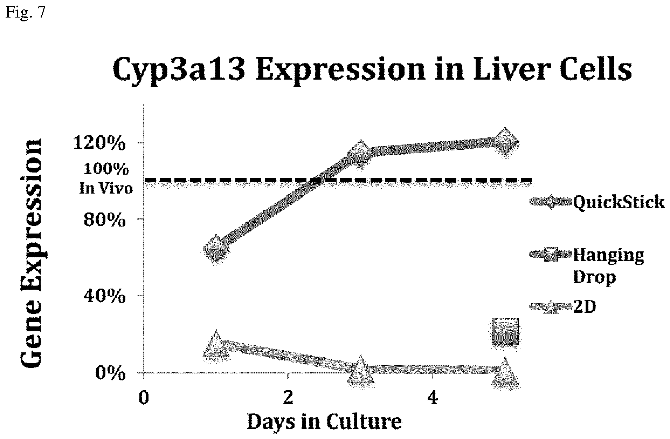

Hepatocytes in QuickStick microtissues displayed 70% expression of Cyp3a13 day 1 in culture compared to freshly isolated hepatocytes, but the expression level increased to values at and above 100% by days 5 and 7. Hanging drop microtissues could only be analyzed earliest at day 5 because they take much longer to form and their day 1 value was diminished to 20% of the normal expression level. It is known that hepatocytes increase the expression of these enzymes over time in hanging drop culture (Messner et al., 2013. "Multi-cell type human liver microtissues for hepatoxicity testing." Archives of Toxicology. Springer-Verlag. 209-213.). As a control, hepatocytes were seeded in 2D in a standard 6-well plate where their expression of cyp31a13 started very low and diminished to 0 by day 3 (see Figure 7).

### Example 4: Mechanical properties of microtissue obtained by QuickStick

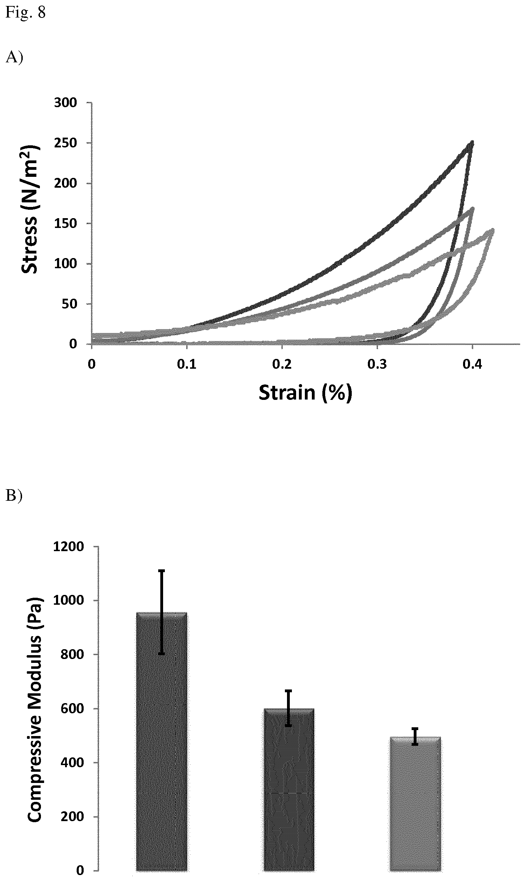

Microtissue mechanical properties can also be tuned by selection of appropriate crosslinking agents. QuickStick microtissues of mesenchymal stem cells were seeded with crosslinking between sChi and either oxidized HA (oxHA), oxidized (oxCS), or oxidized alginate (oxAlg). and the microtissue compressive moduli were calculated on a texture analyzer probe. Stiffness of the microtissue could be correlated to varying the crosslinking material (see also Figure 8).

### Example 5: Patterning of microtissue obtained by QuickStick

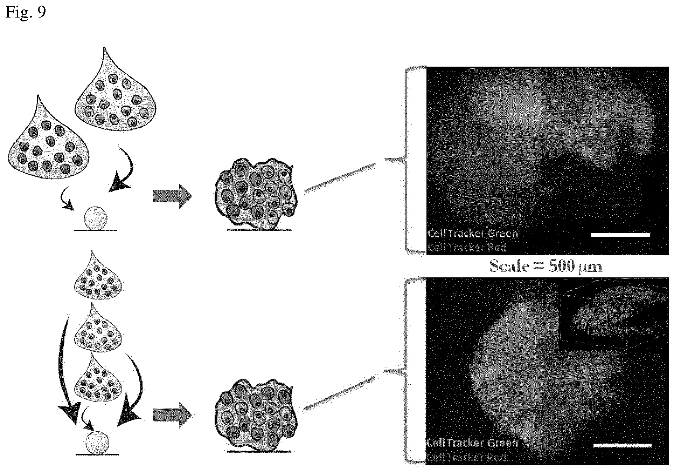

Rapid patterning of different cell populations in a microtissue network was achieved by alternating mixing of different cell populations suspended in sChi. MSCs were separated and labeled with either CellTracker green (CMFDA) or CellTracker red (CMPTX) molecules commercially available from Life Technologies. Sequential pipetting of the cells already suspended in sChi into droplets of oxAlg results in local crosslinking which enables distinct localization of cells from the different populations in specific regions within the microtissues shortly after seeding (see Figure 9).

### Example 6: Combination of growth factors with microtissue obtained by QuickStick

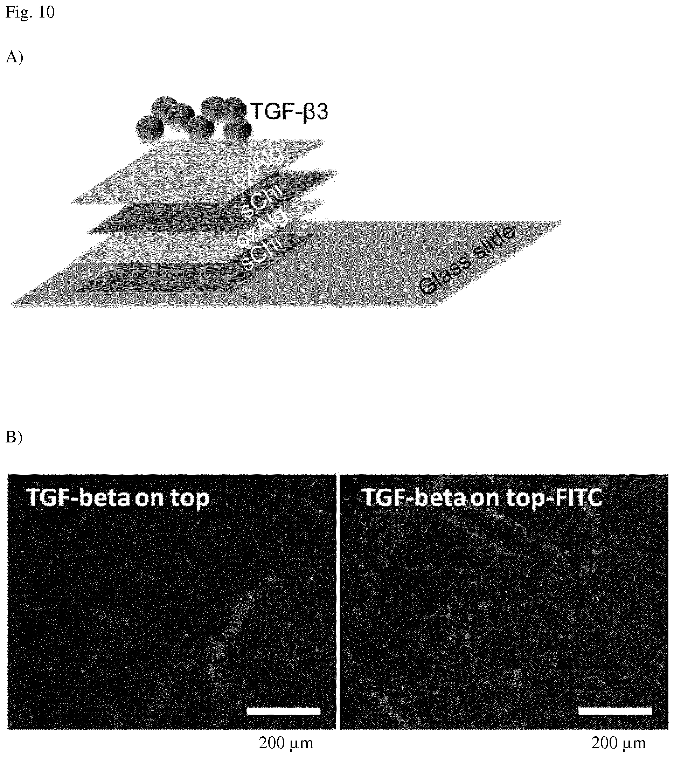

Layers of the sChi and oxAlg were diposed on a standard microscopy slide (see e.g. Figure 10A) and then incubated with the growth factor TGF-beta3 on top for some time. With immunohistochemistry, the binding of the TGF to the polymer matrix was visualized (see Figure 10B).

On the left are layers of sChi and oxAlg that have reduced number of available binding sites for TGF, but still show some presence of the growth factor. On the right, layers were made with lower degree of crosslinking and show much higher binding of the TGF to the polysaccharide network.

### Example 7:

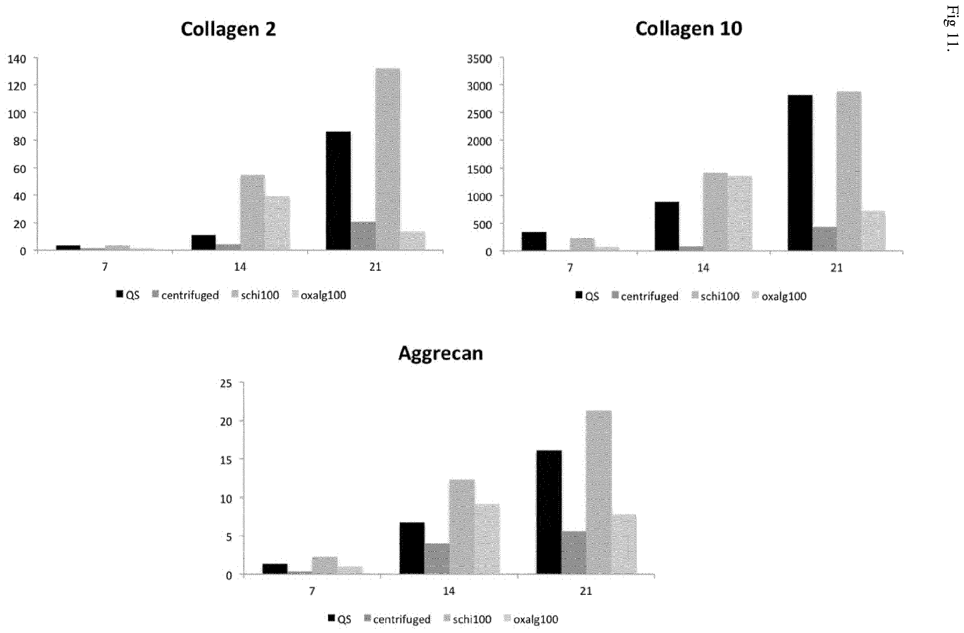

In this study, microtissue were made using MSCs in the QuickStick method without growth factor, state of the art hanging drop and centrifugation, and the growth factor loading with 100ng of TGF either loaded in sChi or oxAlg. For these experiments, TGF was provided in media surrounding QS and centrifuged samples, but not provided in samples that had the TGF loaded inside of the microtissues. The expression of all genes was greatly increased or compatible in the sChi100 samples versus the others. This shows that the growth factor was bound to the QuickStick polymer network and effectively induced the differentiation of the stem cells (see Figure 11).

For loading of the growth factor, TGF-beta 3 was solubilized by a 5mg/mL solution of sChi and a concentration of 10ng/uL. This TGF containing sChi was used to make QuickStick tissues in exactly the same way as normal QuickStick microtissues with the difference being exclusion of the growth factor in media surrounding the tissues for the duration of the experiment.

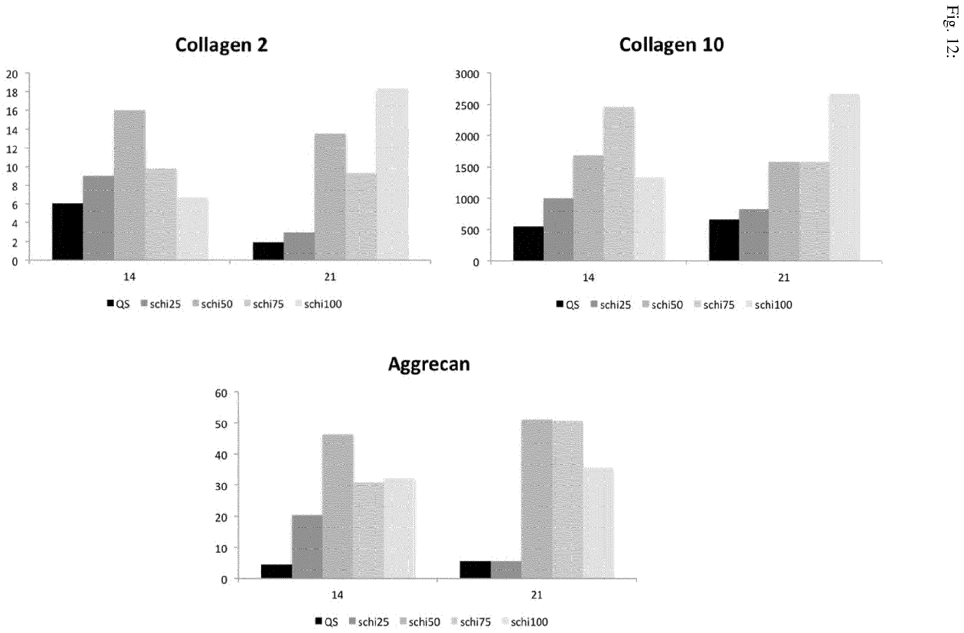

By loading TGF-β3 at different concentrations into sChi (25, 50, 75, and 100 ng TGF-β3), it was shown that the dose of the loaded growth factor effects the outcome of the stem cell differentiation (i.e. that it can be controlled by loading different amounts of the growth factor during microtissue formation) (see Fig. 12).

In the context of the present disclosure, the following embodiments are contemplated:
1. A method of making a three-dimensional microtissue comprising the reaction of at least two cytocompatible polymer solutions, each polymer comprising complementary reactive groups capable of spontaneous reaction, at least one of which solutions additionally contains cells.
2. A method according to embodiment 1, in which the polymer is a biopolymer.
3. A method according to embodiment 2, in which the biopolymer is selected from the group consisting of alginate, alginate sulfate, chondroitin sulfate, dermatin sulfate, hyaluronic acid, cellulose, dextran, poly-1-lysine, chitosan, gelatin, silk and collagen.
4. A method according to embodiment 1, in which the polymer is a synthetic polymer.
5. A method according to embodiment 4, in which the polymer is selected from, or is derived from, the group consisting poly ethylene glycol, poly propylene glycol, polaxomers, polyoxazolines, polyethylenimine, poly vinyl alcohol, poly vinyl acetate, poly methyl vinyl ether-co-maleic anhydride, poly lactide, poly N- isopropylacrylamide, poly glycolic acid, poly methylmethacrylate, poly acrylamide, poly acrylic acid, polyallylamine.
6. A method according to embodiment 1, in which the complementary reactive groups are an aldehyde/amine pairing leading to a Schiff base linkage.
7. A method according to embodiment 1, in which the complementary reactive groups are participants in a Michael addition reaction.
8. A three-dimensional microtissue comprising a crosslinked composition of at least two cytocompatible polymers and cells, the crosslinking being provided by the spontaneous reaction of complementary reactive groups of two types, one of these types being present on each one of the cytocompatible polymers.
9. A screen assay, comprising a microtissue as hereinabove described, in which the cells are adapted to fluoresce on exposure to a stimulus whose detection is desired.
10. A screening assay, comprising a microtissue as hereinabove described, in which the readout of cell viability is measured by MTS assay, alamar blue, MTT assay, or other viability assay where a change in optical density or fluorescence in supernatant is correlated to cell quantity and/or activity.
11. A screening assay, comprising a microtissue as hereinabove described, in which biological changes within the microtissues as a response to stimuli is performed by analytical mass spectroscopy.
12. A screening assay, comprising a microtissue as hereinabove described, in which deposition of endogenous extracellular matrix components are measured by a dye which binds specifically to said extracellular matrix components and produces a colorimetric product that can be measured by spectrophotometry (e.g. dimethylmethylene blue for glycosaminoglycan deposition or hydroxyproline measurements for collagen production).
13. A screening assay, comprising a microtissue hereinabove described, in which changes in gene expression of encapsulated cells is measured by quantitative real time polymerase chain reaction (qPCR).
14. A screening assay, comprising a microtissue hereinabove described, in which changes in cell number within the microtissue is quantified by fluorimetric dye binding to DNA (e.g. PicoGreen).
15. A screening assay, comprising a microtissue hereinabove described, in which morphological changes are monitored online by automated brightfield microscopy and software to calculate morphological features such as optical density, ellipticity, budding, spherical integrity, and diameter.

## Claims

1. A method of making a three-dimensional microtissue containing cells comprising at least the step of:
• Providing at least a first cytocompatible polymer solution comprising at least one first cytocompatible polymer,
• Providing at least a second cytocompatible polymer solution comprising at least one second cytocompatible polymer,
• Reacting said first and second cytocompatible polymer solution with each other to obtain a three-dimensional matrix by forming covalent bonds between said first and second cytocompatible polymer,
wherein the at least first cytocompatible polymer and at least second cytocompatible polymer comprise complementary reactive groups capable of spontaneous reaction, and
wherein at least one of the two cytocompatible polymer solutions additionally contains cells.

2. A method according to claim 1, wherein at least one of the at least first and at least second cytocompatible polymers is selected from the group of biopolymers.

3. A method according to claim 1, wherein at least one of the at least first and at least second cytocompatible polymers is selected from the group of synthetic polymers.

4. A method according to any of claims 1, 2, or 3, wherein the complementary reactive groups are introduced by modification.

5. A method according to any of claims 1, 2, 3, or 4, wherein a biopolymer is selected from or is derived from the group comprising alginate, alginate sulfate, chondroitin sulfate, dermatin sulfate, hyaluronic acid, cellulose, dextran, poly-1-lysine, chitosan, gelatin, silk and collagen, and wherein a synthetic polymer is selected from or is derived from the group comprising poly ethylene glycol, poly propylene glycol, polaxomers, polyoxazolines, polyethylenimine, poly vinyl alcohol, poly vinyl acetate, poly methyl vinyl ether-co-maleic anhydride, poly lactide, poly N- isopropylacrylamide, poly glycolic acid, poly methylmethacrylate, poly acrylamide, poly acrylic acid and polyallylamine.

6. A method according to any of claims 1, 2, 3, 4, or 5, wherein the complementary reactive groups are an aldehyde/amine pairing leading to a Schiff base linkage.

7. A method according to claim 6, wherein a polymer combination of at least succinyl Chitosan and oxidized alginate is provided.

8. A method according to any of claims 1, 2, 3, 4, 5, 6, or 7, wherein the cells are selected from the group comprising mesenchymal stem cells, embryonic stem cells, stromal cells, hepatocytes, neural cells, pancreatic cells, kidney cells, muscle cells, monocytes, endothelial, fibroblasts, epithelial cells, chondrocytes, osteoblasts, osteoclasts and tumor cells.

9. A method according to any of claims 1, 2, 3, 4, 5, 6, 7, or 8, wherein factors that induce and/or influence the development and/or differentiation of cells are added to at least one of the polymer solutions to trigger development and/or differentiation of said cells into downstream lineages.

10. A three-dimensional microtissue obtainable by a method of any of claims 1, 2, 3, 4, 5, 6, 7, 8, or 9.

11. Use of a three-dimensional microtissue obtainable by a method of any of claims 1, 2, 3, 4, 5, 6, 7, 8, or 9 for obtaining tumor, pancreas, liver, bladder, kidney, muscle, cardiac, neural, osteochondral, vasculature, and/or connective tissue microtissues.

12. Use of a three-dimensional microtissue obtainable by a method of any of claims 1, 2, 3, 4, 5, 6, 7, 8, or 9 for screening the influence of microtissues such as tumor, pancreas, liver, bladder, kidney, muscle, cardiac, neural, osteochondral, vasculature, and/or connective tissue microtissues on pharmaceutically active agents.

13. Use of a three-dimensional microtissue obtainable by a method of any of claims 1, 2, 3, 4, 5, 6, 7, 8, or 9 for screening for agents that influence development and/or differentiation of a three-dimensional microtissue.

14. A method of any of claims 1, 2, 3, 4, 5, 6, 7, 8, or 9, a three-dimensional microtissue of claim 10, or use of any of claims 11, 12, or 13, wherein a three-dimensional microtissue of hepatocytes is obtained.

15. A method of any of claims 1, 2, 3, 4, 5, 6, 7, 8, or 9, a three-dimensional microtissue of claim 10, or use of any of claims 11, 12, or 13, wherein a three-dimensional microtissue is obtained and wherein the method makes use of, preferably covalently cross-linked, growth factors such as TGF-β with TGF-β3 being an example and/or cytokines.

## Patentansprüche

1. Verfahren zur Herstellung eines dreidimensionalen Mikrogewebes, das Zellen enthält, umfassend mindestens den Schritt:
• Bereitstellen mindestens einer ersten zytokompatiblen Polymerlösung, umfassend mindestens ein erstes zytokompatibles Polymer,
• Bereitstellen mindestens einer zweiten zytokompatiblen Polymerlösung, umfassend mindestens ein zweites zytokompatibles Polymer,
• Reaktion der ersten und der zweiten zytokompatiblen Polymerlösung miteinander, um durch Bildung von kovalenten Bindungen zwischen dem ersten und dem zweiten zytokompatiblen Polymer eine dreidimensionale Matrix zu erhalten,
wobei das mindestens erste cytokompatible Polymer und das mindestens zweite cytokompatible Polymer komplementäre reaktive Gruppen umfassen, die zur spontanen Reaktion fähig sind, und
wobei mindestens eine der beiden zytokompatiblen Polymerlösungen zusätzlich Zellen enthält.

2. Verfahren nach Anspruch 1, wobei mindestens eines der mindestens ersten und mindestens zweiten cytokompatiblen Polymere aus der Gruppe der Biopolymere ausgewählt ist.

3. Verfahren nach Anspruch 1, wobei mindestens eines der mindestens ersten und mindestens zweiten cytokompatiblen Polymere aus der Gruppe der synthetischen Polymere ausgewählt ist.

4. Verfahren nach einem der Ansprüche 1, 2 oder 3, wobei die komplementären reaktiven Gruppen durch Modifikation eingeführt sind.

5. Verfahren nach einem der Ansprüche 1, 2, 3 oder 4, wobei ein Biopolymer ausgewählt ist aus oder abgeleitet ist von der Gruppe umfassend Alginat, Alginatsulfat, Chondroitinsulfat, Dermatinsulfat, Hyaluronsäure, Cellulose, Dextran, Poly-L-Lysin, Chitosan, Gelatine, Seide und Kollagen, und wobei ein synthetisches Polymer ausgewählt ist aus oder abgeleitet ist von der Gruppe, umfassend Polyethylenglykol, Polypropylenglykol, Polaxomere, Polyoxazoline, Polyethylenimin, Polyvinylalkohol, Polyvinylacetat, Poly(methylvinylether-co-maleinsäureanhydrid), Polylactid, Poly-N-isopropylacrylamid, Polyglykolsäure, Polymethylmethacrylat, Polyacrylamid, Polyacrylsäure und Polyallylamin.

6. Verfahren nach einem der Ansprüche 1, 2, 3, 4 oder 5, wobei die komplementären reaktiven Gruppen ein Aldehyd/Amin-Paar sind, das zu einer Schiffschen Base-Verknüpfung führt.

7. Verfahren nach Anspruch 6, wobei eine Polymerkombination von mindestens Succinyl-Chitosan und oxidiertem Alginat bereitgestellt wird.

8. Verfahren nach einem der Ansprüche 1, 2, 3, 4, 5, 6 oder 7, wobei die Zellen ausgewählt sind aus der Gruppe umfassend mesenchymale Stammzellen, embryonale Stammzellen, Stromazellen, Hepatozyten, Nervenzellen, Pankreaszellen, Nierenzellen, Muskelzellen, Monozyten, Endothel-, Fibroblasten, Epithelzellen, Chondrozyten, Osteoblasten, Osteoklasten und Tumorzellen.

9. Verfahren nach einem der Ansprüche 1, 2, 3, 4, 5, 6, 7 oder 8, wobei mindestens einer der Polymerlösungen Faktoren hinzugefügt sind, die die Entwicklung und/oder Differenzierung von Zellen induzieren und/oder beeinflussen, um die Entwicklung und/oder Differenzierung der Zellen in nachfolgende Linien auszulösen.

10. Dreidimensionales Mikrogewebe, erhältlich durch ein Verfahren nach einem der Ansprüche 1, 2, 3, 4, 5, 6, 7, 8 oder 9.

11. Verwendung eines dreidimensionalen Mikrogewebes, das durch ein Verfahren nach einem der Ansprüche 1, 2, 3, 4, 5, 6, 7, 8 oder 9 erhältlich ist, um Tumor-, Pankreas-, Leber-, Blasen-, Nieren-, Muskel-, Herz-, Nerven-, Osteochondral-, Vaskular- und/oder Bindegewebs-Mikrogewebe zu erhalten.

12. Verwendung eines dreidimensionalen Mikrogewebes, das durch ein Verfahren nach einem der Ansprüche 1, 2, 3, 4, 5, 6, 7, 8 oder 9 erhältlich ist, zum Screening des Einflusses von Mikrogeweben wie Tumor-, Pankreas-, Leber-, Blasen-, Nieren-, Muskel-, Herz-, Nerven-, Osteochondral-, Vaskular- und/oder Bindegewebs-Mikrogeweben auf pharmazeutische Wirkstoffe.

13. Verwendung eines dreidimensionalen Mikrogewebes, das durch ein Verfahren nach einem der Ansprüche 1, 2, 3, 4, 5, 6, 7, 8 oder 9 erhältlich ist, zum Screening nach Mitteln, die die Entwicklung und/oder Differenzierung eines dreidimensionalen Mikrogewebes beeinflussen.

14. Verfahren nach einem der Ansprüche 1, 2, 3, 4, 5, 6, 7, 8 oder 9, ein dreidimensionales Mikrogewebe nach Anspruch 10 oder Verwendung nach einem der Ansprüche 11, 12 oder 13, wobei ein dreidimensionales Hepatozyten-Mikrogewebe erhalten wird.

15. Verfahren nach einem der Ansprüche 1, 2, 3, 4, 5, 6, 7, 8 oder 9, ein dreidimensionales Mikrogewebe nach Anspruch 10 oder Verwendung nach einem der Ansprüche 11, 12 oder 13, wobei ein dreidimensionales Mikrogewebe erhalten wird, und wobei das Verfahren dabei, vorzugsweise kovalent vernetzte, Wachstumsfaktoren wie TGF-β, beispielsweise TGF-β3, und/oder Cytokine nutzt.

## Revendications

1. Procédé de fabrication de microtissus en trois dimensions contenant des cellules comprenant au moins l'étape de :
• mise à disposition au moins d'une première solution de polymère cytocompatible comprenant au moins un premier polymère cytocompatible,
• mise à disposition au moins d'une seconde solution de polymère cytocompatible comprenant au moins un second polymère cytocompatible,
• réaction de ladite première et de ladite seconde solution de polymère cytocompatible l'une avec l'autre pour obtenir une matrice en trois dimensions par la formation de liaisons covalentes entre ledit premier et ledit second polymère cytocompatible,
dans lequel l'au moins premier polymère cytocompatible et l'au moins second polymère cytocompatible comprennent des groupements réactifs complémentaires capables de réagir spontanément, et
dans lequel au moins l'une des deux solutions de polymère cytocompatible contient par ailleurs les cellules.

2. Procédé selon la revendication 1, dans lequel au moins un parmi l'au moins premier et l'au moins second polymère cytocompatible est choisi parmi le groupe des biopolymères.

3. Procédé selon la revendication 1, dans lequel au moins un parmi l'au moins premier et l'au moins second polymère cytocompatible est choisi parmi le groupe des polymères synthétiques.

4. Procédé selon l'une quelconque des revendications 1, 2 ou 3, dans lequel les groupements réactifs complémentaires sont introduits par modification.

5. Procédé selon l'une quelconque des revendications 1, 2, 3 ou 4, dans lequel un biopolymère est choisi parmi ou est dérivé à partir du groupe comprenant l'alginate, le sulfate d'alginate, le sulfate de chondroïtine, le sulfate de dermatine, l'acide hyaluronique, la cellulose, le dextrane, la poly-1-lysine, le chitosane, la gélatine, la soie et le collagène, et dans lequel un polymère synthétique est choisi parmi ou est dérivé à partir du groupe comprenant le polyéthylène glycol, le polypropylène glycol, les polaxomères, les polyoxazolines, la polyéthylène imine, l'alcool polyvinylique, l'acétate de polyvinyle, le copolymère de l'éther méthylvinylique et de l'anhydride maléique, le polylactide, le poly(N- isopropylacrylamide), l'acide polyglycolique, le polyméthacrylate de méthyle, le polyacrylamide, l'acide polyacrylique, et la polyallylamine.

6. Procédé selon l'une quelconque des revendications 1, 2, 3, 4 ou 5, dans lequel les groupements réactifs complémentaires sont un appariement aldéhyde / amine conduisant à une liaison d'une base de Schiff.

7. Procédé selon la revendication 6, dans lequel une combinaison polymère d'au moins de succinyl chitosane et d'alginate oxydé est proposée.

8. Procédé selon l'une quelconque des revendications 1, 2, 3, 4, 5, 6 ou 7, dans lequel les cellules sont choisies parmi le groupe comprenant les cellules souches mésenchymateuses, les cellules souches embryonnaires, les cellules stromales, les hépatocytes, les cellules neurales, les cellules pancréatiques, les cellules rénales, les cellules musculaires, les monocytes, les endothéliales, les fibroblastes, les cellules épithéliales, les chondrocytes, les ostéoblastes, les ostéoclastes et les cellules tumorales.

9. Procédé selon l'une quelconque des revendications 1, 2, 3, 4, 5, 6, 7 ou 8, dans lequel les facteurs qui induisent et / ou influencent le développement et / ou la différenciation des cellules, sont ajoutés à au moins l'une des solutions de polymère pour déclencher le développement et / ou la différenciation desdites cellules dans les lignées en aval.

10. Microtissu en trois dimensions pouvant être obtenu par un procédé selon l'une quelconque des revendications 1, 2, 3, 4, 5, 6, 7, 8 ou 9.

11. Utilisation d'un microtissu en trois dimensions pouvant être obtenu par un procédé selon l'une quelconque des revendications 1, 2, 3, 4, 5, 6, 7, 8 ou 9 pour obtenir des microtissus de tumeur, de pancréas, de foie, de vessie, de rein, de muscle, cardiaques, neuraux, ostéo-cartilagineux, du système vasculaire et / ou du tissu conjonctif.

12. Utilisation d'un microtissu en trois dimensions pouvant être obtenu par un procédé selon l'une quelconque des revendications 1, 2, 3, 4, 5, 6, 7, 8 ou 9 pour le criblage de l'influence de microtissus tels que les microtissus de tumeur, de pancréas, de foie, de vessie, de rein, de muscle, cardiaques, neuraux, ostéo-cartilagineux, du système vasculaire et / ou du tissu conjonctif sur des agents pharmaceutiquement actifs.

13. Utilisation d'un microtissu en trois dimensions pouvant être obtenu par un procédé selon l'une quelconque des revendications 1, 2, 3, 4, 5, 6, 7, 8 ou 9 pour le criblage des agents qui influencent le développement et / ou la différentiation d'un microtissu en trois dimensions.

14. Procédé selon l'une quelconque des revendications 1, 2, 3, 4, 5, 6, 7, 8 ou 9, microtissu en trois dimensions selon la revendication 10, ou utilisation selon l'une quelconque des revendications 11, 12 ou 13, dans lequel un microtissu en trois dimensions d'hépatocytes est obtenu.

15. Procédé selon l'une quelconque des revendications 1, 2, 3, 4, 5, 6, 7, 8 ou 9, microtissu en trois dimensions selon la revendication 10, ou utilisation selon l'une quelconque des revendications 11, 12 ou 13, dans lequel un microtissu en trois dimensions est obtenu et lequel procédé fait usage, de préférence à l'état réticulé par covalence, de facteurs de croissance tels que le TGF-β avec le TGF-β3 qui en est un exemple et / ou de cytokines.
